Europäisches Patentamt

European Patent Office

Office européen des brevets

(19)

(11) Publication number: **0 380 831**
A1

## EUROPEAN PATENT APPLICATION

(12)

(21) Application number: 89300860.7

(22) Date of filing: 30.01.89

(51) Int. Cl.5: **C12P 3/00, C12P 7/24**

(43) Date of publication of application:
08.08.90 Bulletin 90/32

(84) Designated Contracting States:
AT BE CH DE ES FR GB GR IT LI LU NL SE

(71) Applicant: **E.I. DU PONT DE NEMOURS AND COMPANY**
**1007 Market Street**
**Wilmington Delaware 19898(US)**

(72) Inventor: **Anton, David Leroy**
**201 West Pembrey Drive**
**Wilmington Delaware 19803(US)**

(74) Representative: **Woodcraft, David Charles et al**
**BROOKES & MARTIN High Holborn House**
**52/54 High Holborn**
**London, WC1V 6SE(GB)**

(54) Enzymatic production hydrogen peroxide and aldehyde.

(57) An improved process for the enzymatic conversion of alcohol and oxygen to hydrogen peroxide and aldehyde in vitro catalyzed by an alcohol oxidase having no contaminating catalase or catalase-like activity at a temperature lower than 25°C and at a concentration of oxygen in solution of at least one millimolar. A cryosolvent is used at temperatures less than 0°C.

EP 0 380 831 A1

## ENZYMATIC PRODUCTION OF HYDROGEN PEROXIDE AND ALDEHYDE

This invention relates to an improved process for the enzymatic conversion of alcohol and oxygen to hydrogen peroxide and aldehyde in vitro catalyzed by an alcohol oxidase at a temperature lower than 25°C and at a concentration of oxygen in solution of at least one millimolar.

It is known that oxygen and certain alcohols can be enzymatically converted into hydrogen peroxide and corresponding aldehydes in vitro employing various alcohol oxidases as catalyst.

U. S. Patent No. 4,250,261 to Eggeling et al. teaches a process for producing an alcohol oxidase from Hansenula polymorpha yeast. The reference states that it is known in the art that methanol-consuming yeasts such as Candida, Hansenula, Pichia and Torulopsis strains, in growth in the presence of methanol, induce a flavin-adenosine-dinucleotide (FAD) containing alcohol oxidase which catalyzes the oxidation of methanol to formaldehyde.

In the in vivo system, the hydrogen peroxide is rapidly decomposed into water and oxygen by endogenous catalase. Hydrogen peroxide is also a known inactivator of the alcohol oxidase.

U. S. Patent No. 4,503,153 to Geigert, et al. teaches the manufacture of aldehydes by contacting primary alcohols with a reaction mixture consisting of chloroperoxidase and hydrogen peroxide. The reference also discusses, as background, enzymatic oxidation of both primary and secondary alcohols in the presence of co-factors such as NADH and NADPH. In such cases, hydrogen peroxide must be consumed or decomposed to prevent inactivation of the oxidase enzyme.

Bravo et al., at pages 329 to 334, Advanced Biotechnology, 6th 1980 (Published 1981) teach elimination of catalase activity associated with a alcohol oxidase from the yeast Hansenula polymorpha by partial purification through gel filtration chromotography followed by storage of the contaminated alcohol oxidase at a low temperature (6°C) for an extended period. The catalase was totally inactivated while the alcohol oxidase was stable after one week of storage at 6°C. Bravo et al. further teach the use of quinoline for in situ extraction of hydrogen peroxide. Bravo et al. used Hansenula polymorpha as their source for alcohol oxidase because of its known higher resistence towards hydrogen peroxide inactivation. The reference also indicates that methanol protects the enzyme against inactivation by hydrogen peroxide.

Thus, to establish a viable enzymatic process for the coproduction of aldehyde and hydrogen peroxide, references have taught that the endogenous catalase or catalase-like activities associated with cell free extract must be removed or inactivated and the hydrogen peroxide must be quickly removed from the reaction mass.

To determine whether the process is commercially attractive, one should focus attention on the hydrogen peroxide produced and its effect on the alcohol oxidase. While the production of the aldehyde is important to the economics, it is intrinsically more stable than the hydrogen peroxide and does not have so harmful an effect on the enzyme as the hydrogen peroxide.

To be attractive as a commercial process for the manufacture of hydrogen peroxide and aldehyde, the following are important. The hydrogen peroxide should be produced in about equimolar quantities with the aldehyde so as to get the highest possible yield based on the alcohol feed. The concentration of the hydrogen peroxide produced in the reactor should be as high as possible, preferably greater than 3 weight % and more preferably greater than 5 weight %, so as to facilitate separation and purification of the hydrogen peroxide. The decomposition of the hydrogen perioxide should be minimized. The enzyme should be as active as possible, preferably producing greater than 500 grams of hydrogen perioxide per gram of active enzyme so as to minimize the quantity of purified oxidase needed and to reduce enzyme handling cost.

The process of this invention is intended to accomplish the above aspects so as to make the enzymatic production of aldehyde and hydrogen peroxide from oxygen and alcohol catalyzed by a purified alcohol oxidase commercially attractive.

The process of this invention is an improved enzymatic process for the in vitro conversion of oxygen and an alcohol to hydrogen peroxide and the aldehyde corresponding to the starting alcohol. The reaction is catalyzed by an alcohol oxidase that has no contaminating catalase-like or catalase activity. The oxygen dissolved in the reaction solution, which comprises the starting alcohol, the alcohol oxidase, the aldehyde and hydrogen peroxide products, and pH buffers and cryosolvents as necessary, is maintained at a concentration of at least one millimolar (one millimole per liter of reaction solution). The temperature of the reaction is lowered to less than 25°C and preferably less than 0°C.

As the temperature is lowered, a cryosolvent will be needed to keep the reaction solution fluid. Fink et al. discuss cryoenzymology in "Cryoenzymology: The Study of Enzyme Catalysis at Subzero Temperatures", Methods in Enzymology 63, (1979) Chapter 13, pages 336-370, which is incorporated by reference. They set forth the means of selecting the proper cryosolvent that does not have an adverse effect on the catalytic and structural properties of the enzyme. Preferred cryosolvents are aqueous-organic solvent systems. Methanol-based cryosolvents are preferred because of their low viscosity so long as the protein is not too sensitive to methanol.

One skilled in the art would select an aqueous-methanol solution as the cryosolvent in the case of a alcohol oxidase particularly when the starting alcohol is methanol. It follows that, for other starting alcohols, the starting alcohol mixed with water would be the preferred cryosolvent. Other cryosolvents that could be used in mixture with water are ethylene glycol, glycerol, dimethylsulfoxide and dimethylformamide.

It is known that the dielectric constant is critical for many biochemical systems since it determines the interaction between the charged species. The addition of most organic solvents to water causes a drop in dielectric constant, which can be reversed by lowering the temperature. In the process of this invention, the preferred dielectric constant is about 80, that of water at 20°C. Thus, preferably, as the temperature is lowered, the quantity of cryosolvent will be increased sufficiently to cause the dielectric constant of the reaction solution to be from about 70 to 90 and preferably about 80. For example, the dielectric constant of a 50:50 methanol: water solution at -32°C would be about 80.

As temperature decreases, one would expect the rate of enzyme-catalyzed reaction as well as the rate of enzyme inactivation to decrease in accord with the Arrhenius equation.

The process of this invention derives from the finding that the rate of enzyme-catalyzed reaction and the rate of enzyme inactivation vary differently with temperature. The process enzymatically converts oxygen and an alcohol in vitro to hydrogen peroxide and the aldehyde corresponding to the starting alcohol employing a purified alcohol oxidase as the catalyst. A purified alcohol oxidase is one that has no contaminating catalase-like or catalase activity. The concentration of the oxygen dissolved in the reaction solution is maintained at least one millimolar, the temperature is lowered to less than 25°C and preferably less than 0°C, and a cryosolvent is added as needed to keep the reaction solution fluid. Preferably the temperature and the cryosolvent concentration are adjusted so as to maintain a dielectric constant of the reaction solution at about 70 to 90, preferably about 80, the dielectric constant of water at 20°C.

Figures

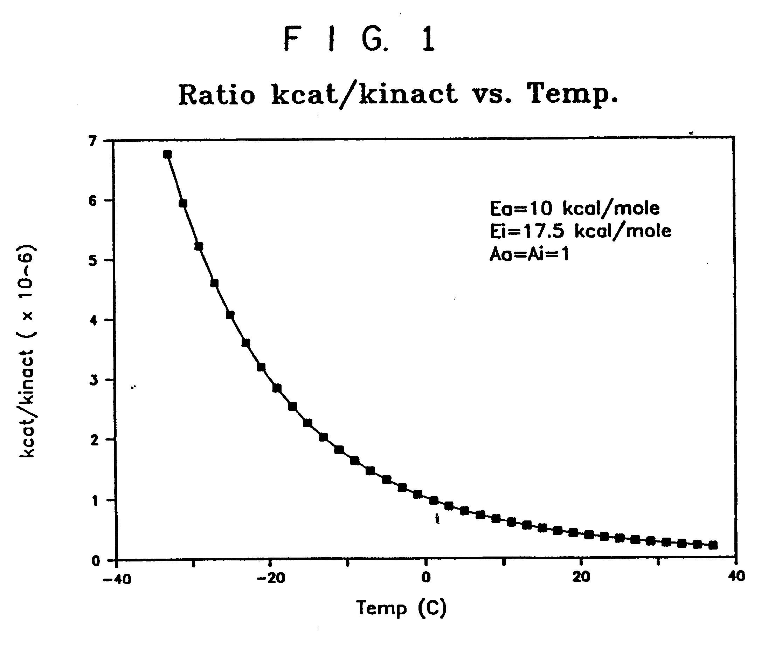

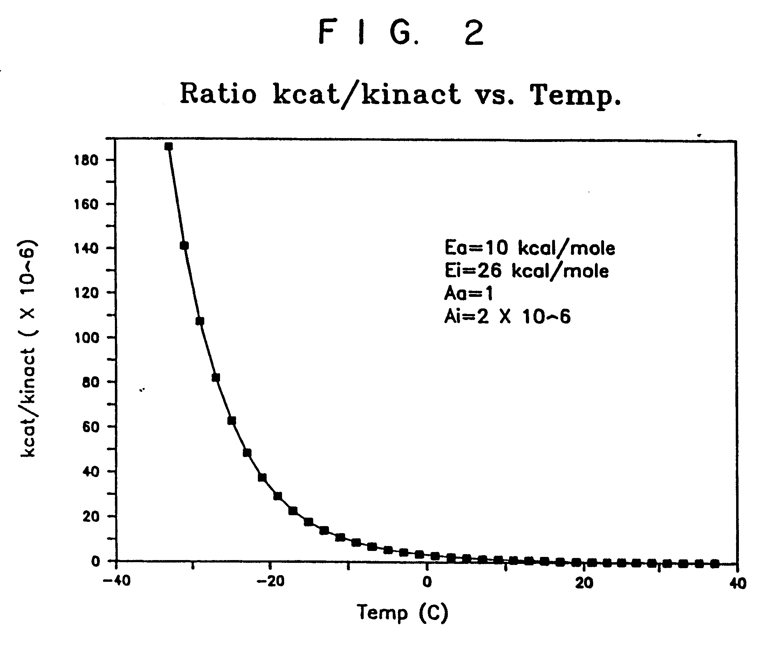

Figure 1 and 2 are plots of the ratio of the rate of reaction to the rate of inactivation of the oxidase versus temperature based on the Arrhenius equation for Examples 3 and 4, respectively.

Detailed Description of the Invention

This invention is an improved process for the enzymatic reaction of oxygen and an alcohol in vitro to produce hydrogen peroxide and an aldehyde corresponding to the starting alcohol. The process employs an alcohol oxidase that has been purified, that is, an alcohol oxidase with no contaminating catalase-like or catalase activity. The improvement comprises operating at a reduced temperature and at an oxygen concentration of at least one millimolar (one millimole per liter of reaction solution).

It has been found that the rate of enzyme-catalyzed reaction (kcat) and the rate of enzyme inactivation, as defined by the following Arrhenius equations, vary differently with temperature:

$$kcat = A_a e^{-Ea/RT} \text{ and } kinact = A_i e^{-Ei/RT},$$

wherein $A_a$ and $A_i$ are pre-exponential constants, Ea is the activation energy, Ei is the inactivation energy, R is the gas constant and T is temperature in degrees Kelvin. Put another way, the energy of activation is less than the energy of inactivation. Thus, lowering the temperature reduces the rate of inactivation faster than the rate of enzyme-catalyzed reaction.

The reaction solution comprises the starting alcohol, the purified alcohol oxidase, dissolved oxygen, the hydrogen peroxide and aldehyde products, and pH buffers and cryosolvents as necessary.

The preferred starting alcohol is methanol. Other suitable alcohols are lower alkyl and alkenyl alcohols having up to four (4) carbon atoms.

The preferred alcohol oxidase is produced from Hansenula polymorpha yeast using known technology. Typically, the yeast cells are disrupted and the extract is purified by known techniques such as ion exchange or gel filtration chromotography. The catalase endogenous to the cell can be totally inactivated by storing the extract at a low temperature (6°C, for example) for an extended period (one week, for example). Other techniques known in the art may be used.

The preferred concentration of alcohol oxidase in the solution depends on the activity of the enzyme and the ability to obtain intimate contact of the enzyme with the substrates in the reaction solution. The lower preferred concentration for a commercially attractive process will depend primarily on the activity of the enzyme and the upper preferred concentration will depend primarily on the ability to maintain contact of the enzyme with the substrates. In the case of free enzyme, the upper preferred concentration will depend on solubility. In the case of immobilized enzyme, that is enzyme attached to a solid matrix, the upper preferred concentration will depend on the ability to maintain the enzyme in suspension. Activity can be measured in terms of units (U), where a unit is the amount of enzyme that will convert 1 micromole of substrate to products under the conditions of operation. At higher activity, lower concentration of alcohol-oxidase will be needed. The milligrams per liter of reaction solution should not exceed the solubility limit for free enzyme at the temperature of operation, since rate would be limited by enzyme not being in solution. For immobilized enzyme, the milligrams per liter should not exceed the level that can be kept in suspension. Preferably, the concentration and activity should be that necessary to produce at least 0.002 moles hydrogen peroxide per liter per minute.

The concentration of the oxygen dissolved in the reaction solution is preferably at least one millimolar. More preferably, the concentration of oxygen should be greater than 2 millimolar. To achieve the preferred concentrations, the oxygen pressure can be controlled based on the temperature of operation, since the solubility of oxygen will increase as the temperature is decreased. At one atmosphere and $0^\circ$C, the solubility of oxygen in water would exceed 2 millimolar. At one atmosphere and $25^\circ$C, the solubility would be about 1.3 millimolar.

The preferred oxygen pressure is at least one atmosphere. Less than one atmosphere is within the scope of the invention so long as the temperature is low enough to assure a one millimolar concentration of the oxygen in the reaction solution. Pressure greater than one atmosphere are satisfactory so long as it is not so high as to damage the enzyme. Preferred oxygen pressure is less than 10 atmospheres.

The temperature of operation should be less than $25^\circ$C and preferably less than $0^\circ$C. Particularly at temperatures lower than $0^\circ$C, a cryosolvent that does not adversely effect the catalytic and structural properties of the enzyme can be added. Theoretically, the minimum temperature of operation is dependent on the freezing point of the reaction solution which is depressed by the cryosolvent. The preferred temperature is the temperature at which the reaction solution would have a dielectric constant of from about 70 to 90, preferably about 80. More preferably, the temperature will also be such that the enzyme-catalyzed rate of reaction is sufficiently high to be attractive as a commercial process.

The preferred cryosolvent is an aqueous-organic solvent system. Particularly when the starting alcohol is methanol, the preferred cryosolvent would be an aqueous-methanol system. The concentration of methanol preferably would be that concentration that would provide a dielectric constant of the reaction solution of from about 70 to 90, preferably about 80 at the temperature of operation. For example, a concentration of a 50:50 aqueous solution of methanol at a temperature of operation of $-32^\circ$C where the dielectric constant would be about 80 would be preferred.

Other preferred cryosolvents would be the aqueous-alcohol systems where the alcohol is the same as the starting alcohol. Other cryosolvents that can be mixed with water are ethylene glycol, glycerol, dimethylsulfoxide and dimethylformamide. Those skilled in the art will be able to determine by known techniques the proper cryosolvent that does not adversely effect the catalytic or structural properties of the enzyme.

It is preferred to maintain the pH of the reaction solution at about pH 6 to pH 8. Preferred pH buffers are phosphates, ammonium bicarbonate, imidazole, and cacodylates.

## Examples

The following examples are illustrative and are not intended to be limiting. Example 1 and 2 are actual examples run at $37^\circ$C and $25^\circ$C, respectively. Examples 3 and 4 are calculations based on the ratio of rates of reaction (kcat) and rates of inactivation (kinact) described by the Arrhenius equations:

$$kcat = A_a e^{-Ea/RT} \text{ and}$$
$$kinact = A_i e^{-Ei/RT},$$

wherein $A_a$ and $A_i$ are pre-exponential constants, Ea is the activation energy, Ei is the inactivation energy, R is the gas constant and T is temperature in degrees Kelvin. Ea for most enzymes has been found to be between 6 and 20 kilocalories per mole (kcal/mol).

## Example 1

A reaction solution containing 100 microliters of an enzyme solution (containing 0.84 milligrams per milliliter (mg/ml) of fast protein liquid chromotography (FPLC) purified alcohol oxidase obtained from a Hansenula polymorpha yeast, 50 millimolar potassium phosphate and 0.5 molar potassium chloride), 1.5 milliliters of 0.1 molar potassium phosphate buffer (pH 7.5), and 10 microliters of methanol was placed into a quartz cuvette. The reaction solution was incubated at $37^\circ$C while bubbling oxygen at 5 pounds per square inch gauge (psig) through the top of the solution in such a way as to avoid having the bubbles pass through the light beam of the spectrophotometer used to monitor the reaction. Hydrogen peroxide production was measured by absorption at 240 nanometers at which hydrogen peroxide has a molar extinction coefficient ($E_{240}$) of 40 per mole per centimeter (40 $M^{-1}cm^{-1}$). The final yield was about 20 micromoles hydrogen peroxide.

## Example 2

A reaction solution containing 100 microliters of the enzyme solution used in Example 1, 1.0 milliliters of 0.1 molar potassium phosphate buffer (pH 7.5), and 10 microliters of methanol was placed into a stirred "Amicon" ultrafiltration cell with gelbond in place of the membrane. The reaction solution was incubated at 25°C while stirring and maintaining oxygen in the sealed vessel at 5 pounds per square inch gauge (psig). Aliquots were removed throughout the run through a sampling port without loss of pressure in the cell. The samples were analyzed for hydrogen peroxide using peroxidase coupled oxidation of o-dianisidine which gives an absorbance change at 460 nanometers. Standard curves were prepared using hydrogen peroxide solutions that had been quantified by using the adsorption at 240 nanometers ($E_{240}$ = 40 $M^{-1}cm^{-1}$). Approximately 50 microliters acidified with 5 microliters of 4 normal hydrochloric acid were used in this analysis for hydrogen peroxide. The final yield was about 56 micromoles hydrogen peroxide.

## Example 3

The data determined in Examples 1 and 2 were compared to the yield increase that would be predicted by the ratio of kcat to kinact as described above. Aa and Ai were assumed each equal one (1). Ea was assumed to be 10 kilocalories per mole (kcal/mol). Ei was calculated based on the ratio of kcat to kinact which was measured at 25°C and found to be 2.8 X $10^5$. The calculated value of Ei was 17.5 kcal/mol. Figure 1 is a plot of the resulting kcat/kinact versus temperature. This model predicts a 1.6 fold increase in yield as the temperature decreases from 37 to 25°C. This compares to the experimental increase of about 2.8. At 0°C, the model would predict a further 3 fold increase in yield over that at 25°C. At -32°C, a further 20 fold increase over the yield at 25°C would be predicted.

## Example 4

The increase-in-yield data determined from Examples 1 and 2 were used to calculate, by trial and error, Ei and Ai asuming Ea was 10kcal/mol, Aa was 1, and using the measured value of kcat/kinact at 25°C (2.8 X $10^5$). Ei was calculated to be 26 kcal/mol and Ai was calculated to be 2 X $10^6$. Figure 2 is a plot of the resulting ratio of kcat to kinact versus temperature. This model predicts a 12 fold increase in yield at 0°C and a 600 fold increase in yield at -32°C over that at 25°C.

## Claims

1. A process for the enzymatic conversion of a starting alcohol and oxygen to hydrogen peroxide and aldehyde in vitro catalyzed by an alcohol oxidase that has no contaminating catalase or catalase-like activity comprising operating at a temperature lower than 25°C and maintaining a reaction solution with at least one millimole of dissolved oxygen per liter of reaction solution.

2. A process according to claim 1 wherein the temperature is less than 0°C comprising the further addition of a sufficient quantity and concentration of a cryosolvent to cause the dielectric constant of the reaction solution to be between about 70 and 90.

3. A process according to claim 2 wherein the starting alcohol is methanol and the cryosolvent is an aqueous-medium solution.

4. A process according to claim 3 wherein a sufficient quantity and concentration of the aqueous-methanol solution is added to cause the dielectric constant to be about 80.

5. A process according to claim 3 or 4 wherein the cryosolvent is a 50:50 solution of water and methanol and the temperature is about -32°C.

6. A process according to claim 1 or 2 wherein the starting alcohol is an alkyl or alkenyl alcohol having up to four carbon atoms.

7. A process according to claim 6 wherein the cryosolvent is an aqueous-alcohol solution, the alcohol being the same alcohol as the starting alcohol.

8. A process according to any one of the preceding claims wherein the alcohol oxidase is produced from Hansenula polymorpha yeast.

# F I G. 1

## Ratio kcat/kinact vs. Temp.

Ea=10 kcal/mole
Ei=17.5 kcal/mole
Aa=Ai=1

EP 0 380 831 A1

# F I G. 2

## Ratio kcat/kinact vs. Temp.

Ea=10 kcal/mole
Ei=26 kcal/mole
Aa=1
Ai=2 X 10~6

kcat/kinact ( X 10~6)

Temp (C)

EP 0 380 831 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 808 879 (AMERICAN BIOGENETICS CORP.) <br> * Claims 1-12,16-19-23; page 25, lines 1-15; page 9, line 20 - page 10, line 3; page 12, lines 10-18; page 17, lines 13-26; page 15, line 34 - page 16, line 7 * <br> --- | 1-8 | C 12 P    3/00 <br> C 12 P    7/24 |
| A | WO-A-8 704 725 (AMERICAN BIOGENETICS CORP.) <br> * Claims 1-20 * <br> ----- | 1-8 | |

TECHNICAL FIELDS SEARCHED (Int. Cl.5)

C 12 P
C 12 N

The present search report has been drawn up for all claims

| Place of search | Date of completion of the search | Examiner |
|---|---|---|
| THE HAGUE | 19-09-1989 | EPAILLARD P.J.H.M. |